# EUROPEAN PATENT SPECIFICATION

(11) **EP 4 485 656 B1**
(45) Date of publication and mention of the grant of the patent: **28.10.2026**
(21) Application number: 23759893.3
(22) Date of filing: 17.02.2023
(51) Int. Cl.: H01M 50/271, G01N 33/66, H01M 50/247, A61B 5/145

(54) **BLOOD GLUCOSE METER**
BLUTZUCKERMESSGERÄT
GLUCOMÈTRE

(30) Priority: 24.02.2022 JP 2022026856
(43) Date of publication of application: 01.01.2025
(73) Proprietor: PHC Holdings Corporation, Tokyo 100-8403 (JP)
(72) Inventor: TANIZAKI, Youichi, Ehime 791-0395 (JP); IKEGAWA, Shugo, Ehime 791-0395 (JP)
(74) Representative: Eisenführ Speiser
(86) International application number: PCT/JP2023/005822
(87) International publication number: WO 2023/162892

(56) References cited:
- EP-A1- 3 012 883
- CN-U- 208 656 838
- FR-A1- 2 945 591
- JP-A- 2001 068 082
- JP-A- 2008 258 004
- JP-A- 2013 050 326
- JP-A- H11 233 090
- JP-U- 3 020 323

## Description

### TECHNICAL FIELD

The present invention relates to a blood glucose meter that measures blood glucose levels.

### BACKGROUND ART

Conventional blood glucose meters generally use a small, replaceable battery, such as a button battery.

When the battery is to be replaced, the battery cover is removed from the main body part, and a new battery is installed in the battery compartment provided to the main body part.

Since button batteries or other such small batteries are sometimes used for this purpose, in view of the risk of accidental swallowing by children, it is preferable for the structure to be such that the battery cannot be easily removed.

For instance, Patent Literature 1 discloses a portable device equipped with a battery that is aesthetically pleasing and that allows the battery cover to be easily opened by smoothly releasing the locked state.

A device according to FR 2 945 591 A1 has a box body on which a spike is positioned, where the body is arranged on a support. The support authorizes vertical displacement of the spike. A projecting part is placed on the support. A groove is formed on a battery compartment. A hole guides and receives the spike. An upper face of the spike forms an angle higher than angle of the groove. The angle unlocks the compartment from the body by any object of diameter lower than diameter of the hole and to translate the compartment from the body.

### CITATION LIST

### PATENT LITERATURE

Patent Literature 1: JP-A 2008-258004

### SUMMARY

### (TECHNICAL PROBLEM)

However, the following problems are encountered with the conventional portable devices mentioned above.

With the portable device disclosed in the above-mentioned publication, when an attempt is made to forcibly open the locked battery cover, if a large force is exerted on the locking structure, and cover may become unlocked, allowing the battery cover to be opened, which poses the risk that a serious accident may occur in which the battery is accidentally swallowed by a child or the like.

It is an object of the present invention to provide a blood glucose meter equipped with a locking mechanism that can boost the locking strength while preventing a child from easily removing the battery cover, for example.

### (SOLUTION TO PROBLEM)

The blood glucose meter according to the invention is defined in claim 1. Preferred embodiments are defined in the dependent claims.

The blood glucose meter comprises a main body part, a sensor mounting part, a measurement unit, a battery compartment, a battery cover, an elastic deformation part, a lock portion, and a slide part. The sensor mounting part is provided to the main body part, and a blood glucose level measurement sensor is mounted thereon. The measurement unit measures blood deposited on the blood glucose value measurement sensor mounted on the sensor mounting part. The battery compartment holds a battery that supplies power to the measurement unit. The battery cover is removably attached to the main body part so as to cover the battery compartment, and has an opening into which a jig is inserted during removal from the main body part. The elastic deformation part is provided at a position opposite the battery cover in the main body part and deforms when pressed by the jig inserted through the opening. The lock portion is provided at a position exposed to the outside from the opening, is molded integrally with the elastic deformation part, and unlatches the battery cover from the main body part when pressed by the jig. The slide part is provided to the main body part and slides so as to guide the battery cover in the removal direction when the lock portion is unlatched from the main body part. When the battery cover is slid along the slide part while the battery cover remains latched to the main body part by the lock portion, a force in the compression direction is exerted on the elastic deformation part.

Here, in a blood glucose meter that measures blood glucose level in a state in which a blood glucose level measurement sensor on which the blood of a diabetic patient or the like is deposited has been mounted, and that is powered by a battery held in a battery compartment of a main body part, when opening a battery cover attached to the main body part is opened to replace the battery, for example, a jig is inserted into the opening of the battery cover to deform the elastic deformation part and release the latching by the lock portion, allowing the battery cover to be slid to its open state, and if an attempt is made to forcibly slide the battery cover while latched by the lock portion, a force is exerted on the elastic deformation part in the compression direction.

Consequently, in opening the battery cover, it is necessary to use a jig to press the lock portion through the opening to release the latching, and also to slide the battery cover, and this prevents a child, etc., from accidentally removing the battery cover.

Furthermore, if the battery cover is forcibly slid while still latched to the main body part by the lock portion, a force will be exerted on the elastic deformation part in the compression direction, making it less likely that the elastic deformation part will be damaged.

As a result, it is possible to provide a blood glucose meter equipped with a locking mechanism that can boost latching strength while preventing children from easily removing the battery cover, for example.

The blood glucose meter according to the second invention is the blood glucose meter according to the first invention, further comprising a capillary flow path that is provided so as to surround at least part of the opening in the battery cover, and that guides liquid that has entered through the opening by capillary action to the outside of the main body part.

Consequently, in a configuration in which a battery cover is provided with an opening, liquid such as blood or alcohol clinging near the opening will be guided in the desired direction (to outside the main body part) along the capillary flow path by capillary action. This prevents liquid from entering the battery compartment through the opening.

The blood glucose meter according to the third invention is the blood glucose meter according to the second invention, wherein the capillary flow path is formed as a groove provided between a plurality of ribs provided around the opening.

Consequently, the plurality of ribs provided so as to surround at least a portion of the outer periphery of the opening form capillary flow paths in between the ribs. Therefore, it is possible to prevent liquid adhering to the vicinity of the opening from infiltrating into the battery compartment.

The blood glucose meter according to the fourth invention is the blood glucose meter according to the second or third invention, wherein the capillary flow path is provided on the rear surface side of the battery cover.

Consequently, when liquid clinging near the opening of the battery cover seeps around to the back side, the liquid can be guided in the desired direction (to outside the main body part) by the capillary flow path.

The blood glucose meter according to the fifth invention is the blood glucose meter according to any of the second to fourth inventions, further comprising a liquid guiding flow path that is provided to the main body part and guides the liquid that has come in through the opening to the outside of the main body part.

Consequently, the liquid guided in the desired direction by the capillary flow path provided to the battery cover can be guided to the outside of the main body part by the liquid guiding flow path provided on the main body part side. This avoid problems caused when liquid that has come in through the opening moves into the battery compartment.

The blood glucose meter according to the sixth invention of the invention is the blood glucose meter according to the fifth invention, wherein the liquid guiding flow path is provided to the main body part at a position opposite the capillary flow path.

Consequently, the liquid that has been guided by the capillary flow path provided on the battery cover side moves to the liquid guiding flow path provided to the main body part at a position opposite the capillary flow path, and is moved by the liquid guiding flow path to outside the main body part. This prevent problems attributable to liquid that has come into the main body part through the opening in the battery cover.

The blood glucose meter according to the seventh invention of the present invention is the blood glucose meter according to any of the first to sixth inventions, further comprising a latching hole that is provided to the main body part and is formed along the sliding direction of the battery cover, and a latched part that is provided to the battery cover and is inserted into the latching hole and restricts movement in a direction intersecting the sliding direction when the battery cover is attached to the main body part.

Consequently, when the battery cover is attached to the main body part, the battery cover is prevented from lifting up in the direction intersecting the sliding direction by the latched state between the latching hole and the latched part, and this maintains the locked.

The blood glucose meter according to the eighth invention is the blood glucose meter according to any of the first to seventh inventions, wherein the lock portion is formed such that the end that is farther from the center of deformation when the jig is inserted into the opening and the elastic deformation part is deformed is higher than the end that is closer to the center of deformation.

Consequently, when the elastic deformation part is deformed, the end of the lock portion located farther from the center of deformation is higher than the end on the opposite side (the side nearer the center), and this ensures plenty of room for latching by the lock portion to the inner peripheral surface of the opening in the battery cover, while allowing the battery cover to be easily removed.

The blood glucose meter according to the ninth invention is the blood glucose meter according to any of the first to eighth inventions, wherein the elastic deformation part has a reinforcing rib that is provided at a position close to the lock portion and has a thicker wall than a position farther away from the lock portion.

This increases the rigidity of the elastic deformation part near the lock portion, which prevents damage to the elastic deformation part and also moves the center of deformation away from the lock portion, which prevents the lock portion from easily slipping out of the opening and becoming unlatched.

The blood glucose meter according to the tenth invention is the blood glucose meter according to any of the first to ninth inventions, wherein the lock portion is provided at a position farther to the inside than the outer contour of the main body part, through the opening.

Consequently, the lock portion, which is located deeper than the outer part of the main body part, is less likely to be touched accidentally, which prevents the battery cover from being accidentally unlocked from the main part.

The blood glucose meter according to the eleventh invention is the blood glucose meter according to any of the first to tenth inventions, wherein the opening has a chamfered portion whose diameter increases, at the upper end in the insertion direction of the jig.

Consequently, the chamfered portion forms a circular shape with a larger diameter at the end on the insertion side, so any rod-shaped member with a pointed tip can be easily used as a jig.

The blood glucose meter according to the twelfth invention is the blood glucose meter according to any of the first to eleventh inventions, wherein the opening has a diameter smaller than the size of a child's finger.

Consequently, children can be prevented from accidentally unlatching the battery cover by providing an opening with a diameter smaller than about 1 cm, for example.

The blood glucose meter according to the thirteenth invention is the blood glucose meter according to the first or second invention, wherein the elastic deformation part has a recess formed so as to avoid the position of a screw attached to the main body part.

Consequently, in top view, the elastic deformation part does not end up covering the position of the screw, so the screw can be easily threaded into the screw hole.

The blood glucose meter according to the fourteenth invention is the blood glucose meter according to the first or second invention, wherein the main body part has a latching prong that protrudes from an outer peripheral side of the battery compartment and holds the battery housed in the battery compartment.

Consequently, the battery is effectively prevented from falling out of the battery compartment when the battery cover has been removed.

The blood glucose meter according to the fifteenth invention is the blood glucose meter according to the first or second invention, wherein the elastic deformation part is fixed to the main body part at a plurality of positions that are away from the lock portion.

Consequently, if there is anything other than a screw that interferes with the elastic deformation part or the moisture guiding structure, for example, this configuration allows such structures to be avoided.

### (EFFECTS)

With the blood glucose meter of the present invention, it is possible to provide a blood glucose meter that is equipped with a locking mechanism that is resistant to damage, while preventing a child from easily removing the battery cover, for example.

### BRIEF DESCRIPTION OF THE DRAWINGS

Fig. 1 is an overall oblique view of the configuration of the blood glucose meter in an embodiment of the present invention;
Fig. 2 is a control block diagram of the blood glucose meter in Fig. 1;
Fig. 3A is an oblique view of a state in which a jig has been inserted into the opening of the battery cover to release the lock, and Fig. 3B is an oblique view of the step of sliding and removing the unlocked battery cover of Fig. 3A from the main body part;
Fig. 4 is an oblique view of the state when the battery cover has been removed from the main body part from the state in Fig.3B to expose the button battery;
Fig. 5 is a cross-sectional view of the configuration around the locking mechanism 20 in the locked state in Fig. 3A;
Fig. 6A is a cross-sectional view of the state when the battery cover has been locked to the main body part by the locking mechanism, and Fig. 6B is a cross-sectional view of the state when the jig has been inserted through the opening to release the locking mechanism;
Fig. 7A is an oblique view of the configuration around the lock pin in a state in which the battery cover has been locked to the main body part by the locking mechanism, and Fig. 7B is an oblique view of the configuration around the lock pin in a state in which the locking mechanism has been released;
Fig. 8 is a cross-sectional view of the state when the locking state by the locking mechanism has been released and the battery cover is slid;
Fig. 9A is a plan view of the blood glucose meter, and Fig. 9B is a cross-sectional view along the A-A line in Fig. 9A;
Fig. 10 is a detail view of the X portion in Fig. 9B;
Fig. 11A is an oblique view of the configuration on the main body part side where the battery cover is removed, and Fig. 11B is a detail view of the Y portion in Fig. 11A;
Fig. 12 is a cross-sectional view of a configuration in which the first end side of the lock pin included in the locking mechanism is formed higher;
Fig. 13A is an oblique view of the configuration of the battery cover, and Fig. 13B is an oblique view of the state when Fig. 13A is flipped upside-down;
Fig. 14 is an oblique view of the configuration around the rib group provided on the back side in Fig. 13B;
Fig. 15 is an oblique view of a liquid guide path for discharging to the outside the liquid that has moved along the capillary flow path formed by the rib group in Fig. 14 and the liquid guiding flow path formed by the grooves of the main body part;
Fig. 16 is a cross-sectional view of the liquid guide path for discharging to the outside the liquid that has moved along the capillary flow path formed by the rib group in Fig. 14 and the liquid guiding flow path formed by the grooves of the main body part;
Fig. 17 is an oblique view of the blood glucose meter according to another embodiment of the present invention, and shows the state when the battery cover has been removed from the main body part;
Fig. 18 is a detail view of the configuration around the locking mechanism provided to the blood glucose meter in Fig. 17;
Fig. 19A is a detail view of latching prongs that prevent a battery held in the battery compartment of the main body part from falling out, and Fig. 19B is a detail view of a state in which the latching prongs are holding a battery housed in the battery compartment;
Fig. 20 is a detail view of the locking mechanism provided to the blood glucose meter in Fig. 17, and shows a state in which the lock pin deforms in the direction of biting into the opening of the battery cover as the battery cover is slid in the direction of coming off;
Fig. 21 is an oblique view of the configuration of the battery cover constituting part of the blood glucose meter in Fig. 17; and
Fig. 22 is a cross-sectional view of a liquid guide path for discharging to the outside the liquid that has come into the interior of the main body part in a state in which the battery cover in Fig. 21 has been attached to the main body part.

### DESCRIPTION OF THE EMBODIMENTS

### Embodiment 1

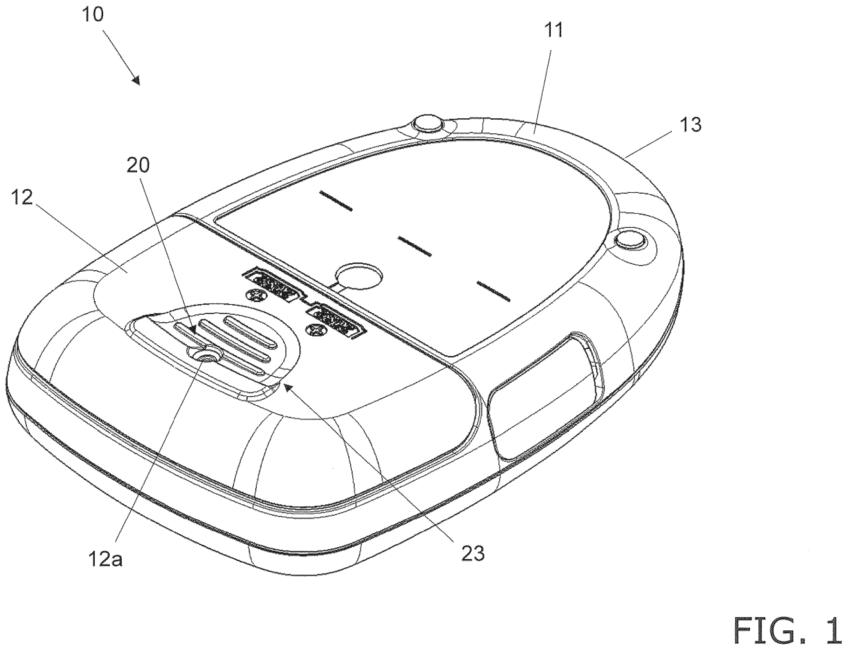

The blood glucose meter 10 according to an embodiment of the present invention will now be described through reference to Figs. 1 to 16.

In this embodiment, some unnecessarily detailed description may be omitted. For example, detailed description of already known facts or redundant description of components that are substantially the same may be omitted. This is to avoid unnecessary repetition in the following description, and facilitate an understanding on the part of a person skilled in the art.

The applicant has provided the appended drawings and the following description so that a person skilled in the art might fully understand this disclosure, but does not intend for these to limit what is discussed in the patent claims.

### (1) Configuration of Blood Glucose Meter 10

The blood glucose meter 10 according to this embodiment is used at home, in hospitals and other such facilities, etc., and measures the blood glucose value (glucose concentration in blood) in a state in which a sensor for measuring blood glucose level, on which the blood of a diabetic patient or other such measurement subject has been deposited, has been inserted into a sensor mounting part 13 provided at the end of a main body part 11.

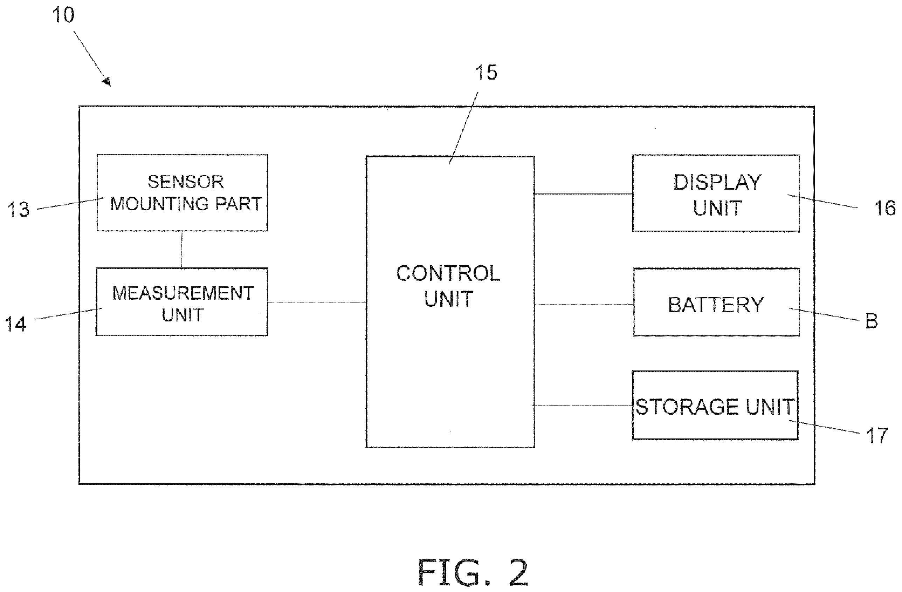

As shown in Figs. 1 and 2, the blood glucose meter 10 comprises the main body part 11, a battery cover 12, the sensor mounting part 13, a measurement unit 14, a control unit 15, a display unit 16, button batteries (batteries) B, a storage unit 17, and a locking mechanism 20.

In the main body part 11, the display unit 16 and so forth are provided on the front side, a battery cover 12 is removably attached to the back side, and the measurement unit 14, the control unit 15, the button batteries (batteries) B, the storage unit, and so forth are provided on the inside.

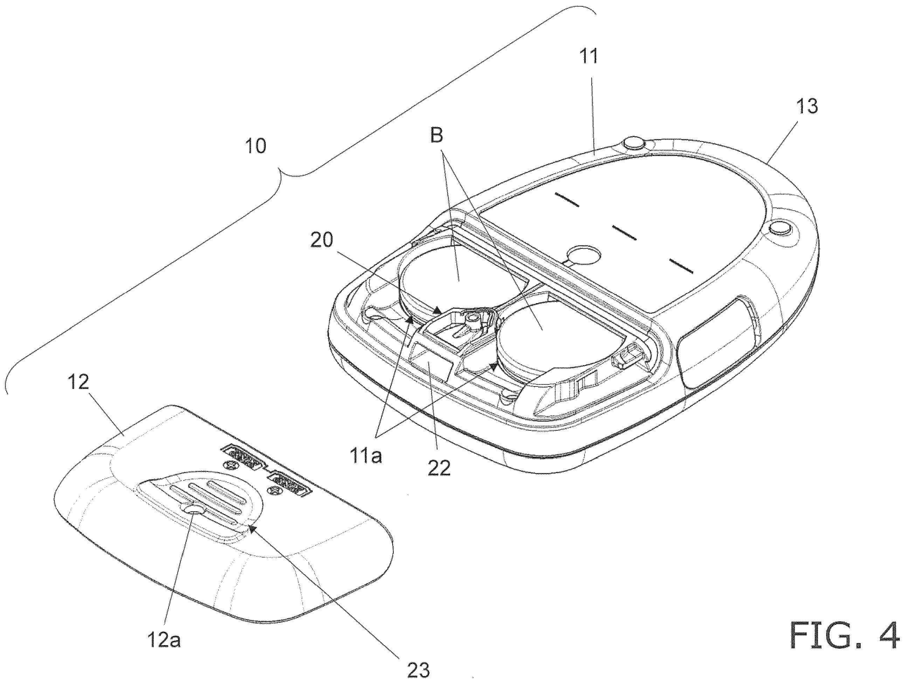

The battery cover 12 is removably attached to the main body part 11 so as to cover a battery compartment 11a (see Fig. 4, etc.) on the main body part 11 side, and when the battery cover 12 is to be removed from the main body part 11, a jig Z (see Fig. (a), etc.) is inserted into the opening 12a.

The sensor mounting part 13 is provided on the end surface of the main body part 11 shown in Fig. 1, and a blood glucose level measurement sensor (not shown) is inserted therein. The sensor mounting part 13 is connected to the measurement unit 14 and is electrically connected to the electrodes of the blood glucose level measurement sensor.

The measurement unit 14 is connected to the control unit 15 and the sensor mounting part 13, and measures the glucose concentration in blood by means of an electrical signal received via the sensor mounting part 13.

More specifically, when blood is deposited on the blood glucose level measurement sensor in a state in which the blood glucose level measurement sensor has been attached to the sensor mounting part 13, the blood glucose level in the blood is measured by the measurement unit 14.

As shown in Fig. 2, the control unit 15 is connected to the measurement unit 14, the display unit 16, the button batteries (batteries) B, and the storage unit 17. The control unit 15 reads various kinds of information (data) stored in the storage unit 17 and executes various kinds of processing using the blood glucose value data measured by the measurement unit 14.

The display unit 16 is disposed on the surface on the opposite side from the main body part 11 as shown in Fig. 1, and performs various displays. As shown in Fig. 2, the display unit 16 is connected to the control unit 15, and displays the measurement results from the measurement unit 14, the analysis results based on these measurement results, the current time, and the like.

The button batteries B are connected to the control unit 15, and are installed in the battery compartment 11a (see Fig. 4, etc.) provided to the main body part 11 as a power source for driving the measurement unit 14, the display unit 16, etc.

The storage unit 17 is connected to the control unit 15 and stores various kinds of information (data), such as blood glucose value data measured by the measurement unit 14, information about the time of measurement, and analysis results based on the measurement results.

A nonvolatile memory such as an EEPROM (electrically erasable programmable read-only memory) can be used as the storage unit 17.

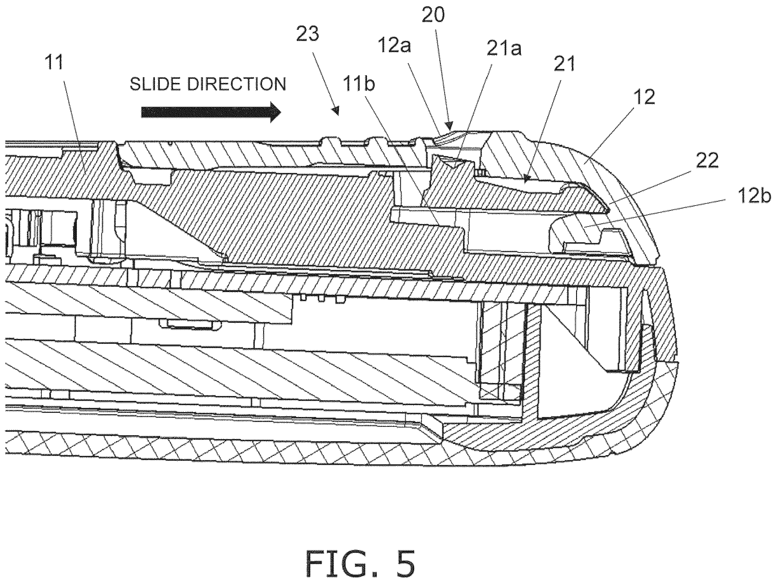

As shown in Fig. 5, the locking mechanism 20 is made up of a hinge 21 and a latching hole 22 provided on the main body part 11 side, and an opening 12a and a latched portion 12b provided on the battery cover 12 side.

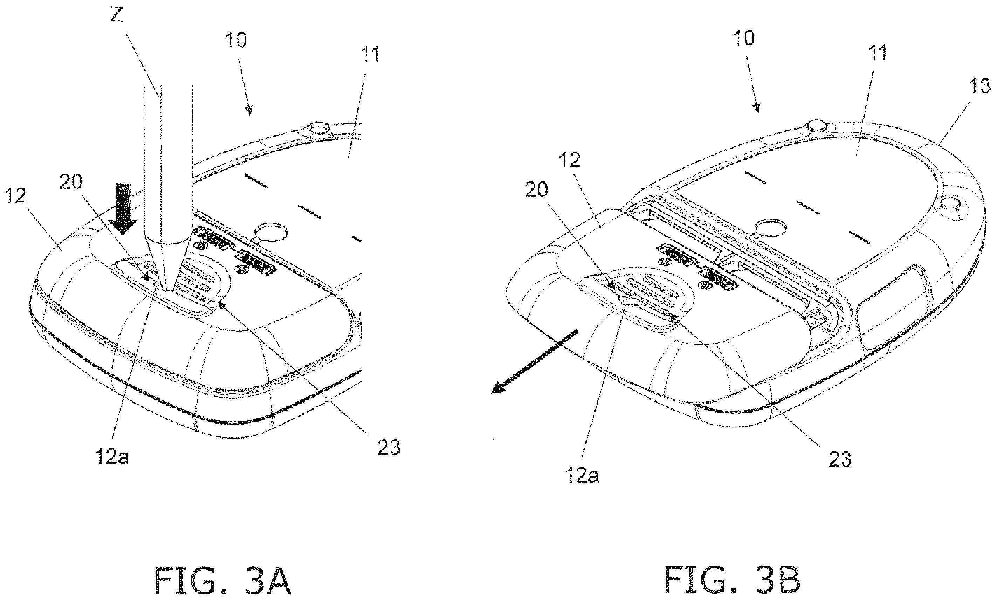

As shown in Fig. 3A, the hinge 21 is elastically deformed when the jig Z is inserted into the opening 12a of the battery cover 12 and pressed downward, which causes part of the hinge 21 (a lock pin 21a; see Fig. 5, etc.) latched in the opening 12a to come out of the opening 12a and releases the locked state.

The lock pin 21a is a substantially cylindrical portion integrally formed so as to protrude upward in the drawing at one end of the hinge 21, and in the locked state, restricts the movement of the battery cover 12 in the sliding direction in a state of being inserted in the opening 12a of the battery cover 12.

The latching hole 22 is a hole made in the end surface of the main body part 11, and is formed along the sliding direction of the battery cover 12.

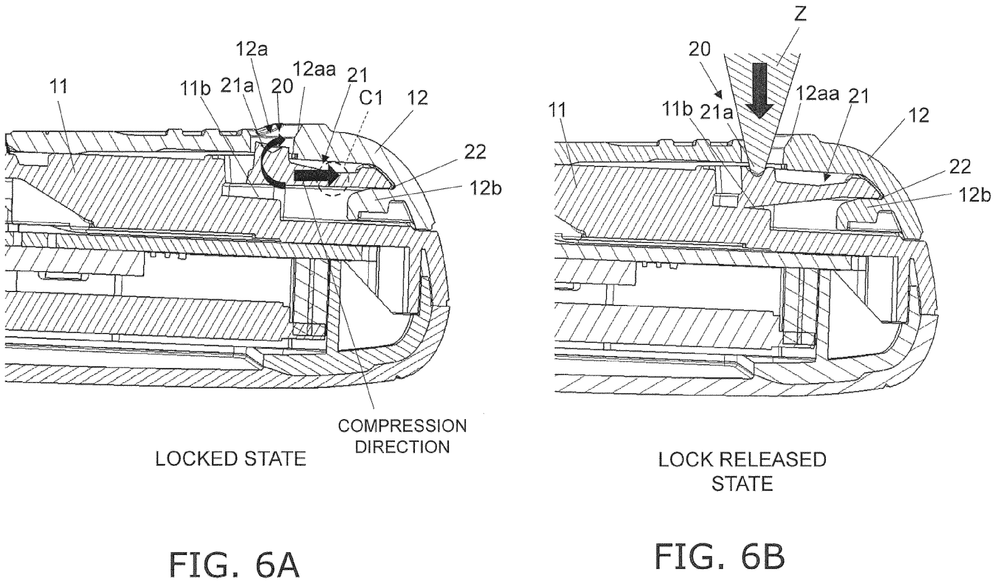

As shown in Fig. 1, etc., the opening 12a is a through-hole formed in the approximate center in the width direction of the battery cover 12, and in the locked state, the lock pin 21a is latched, and when transitioning to an unlocked state, the jig Z is inserted as shown in Fig. 3A. Also, the opening 12a has a C surface (chamfered portion) 12aa (see Figs. 6A and 6B, etc.) whose diameter increases upward when viewed in the insertion direction of the jig Z.

This allows a rod-shaped member that is pointed at the tip to be easily used as the jig Z for unlocking the battery cover 12.

Furthermore, the opening 12a has a diameter whose smallest inside diameter is less than the size of a child's finger.

Therefore, providing an opening having a diameter smaller than about 1 cm, for example, prevents children from accidentally unlocking the battery cover 12.

The latched portion 12b is provided on the inner surface side of the battery cover 12 and is formed so as to protrude in the sliding direction of the battery cover 12. When the battery cover 12 is in its locked state, the latched portion 12b is held in a state of being inserted into the latching hole 22, as shown in Fig. 5.

Consequently, movement of the battery cover 12 in a direction intersecting the sliding direction (upward in Fig. 5) is restricted, and the battery cover 12 is held in place relative to the main body part 11.

### (2) Locking Mechanism 20 of Battery Cover 12

With the blood glucose meter 10 of this embodiment, when the button batteries B are installed in the battery compartment 11a of the main body part 11, or when the button batteries B are replaced, the jig Z with a pointed tip is inserted into the opening 12a and pressed as shown in Fig. 3A in order to remove the battery cover provided on the back side of the main body part 11. Then, as shown in Fig. 3B, the user places a finger on the finger pad 23, which includes a depression on the surface of the battery cover 12 and three ribs of varying length, and slides the battery cover 12 in the direction of the arrow in the drawing, which releases the locking of the battery cover 12 by the locking mechanism 20.

Consequently, as shown in Fig. 4, the battery cover 12 can be removed from the main body part 11 and the button batteries B can be installed in the battery compartment 11a of the main body part 11, or can be replaced with new button batteries B.

At this time, as shown in Fig. 4, the battery cover 12 is unlocked by the locking mechanism 20 provided near the battery compartment 11a of the main body part 11, and is removed from the main body part 11.

That is, in removing the battery cover 12 from the main body part 11, it is necessary to use the jig Z shown in Fig. 3A to press the lock pin 21a (see Fig. 5) from the opening 12a to release the lock, and slide the battery cover 12. Therefore, if a child, etc., should handle the blood glucose meter 10, for example, this configuration prevents the accidental removal of the battery cover 12 and swallowing of the button batteries B.

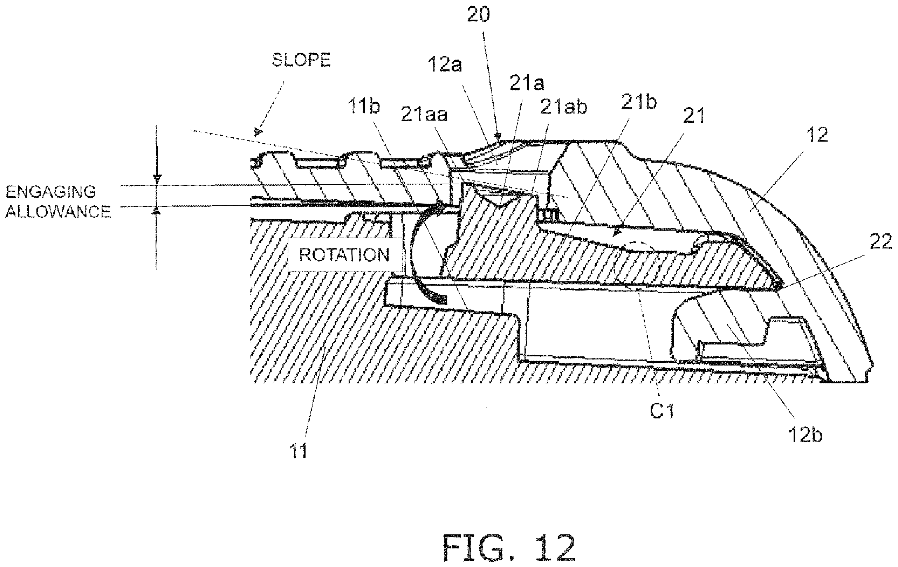

As shown in Figs. 5 and 12, in the locking mechanism 20, the substantially cylindrical lock pin 21a provided on the first end side of the hinge (elastic deformation part) 21 is inserted into an opening 12a of the battery cover 12 and held in that state, thus rendering the battery cover 12 immovable in the sliding direction.

Also, when the battery cover 12 is locked by the locking mechanism 20, movement of the battery cover 12 in a direction intersecting the sliding direction is restricted because the latched portion 12b inserted and latched in the latching hole 22 provided along the sliding direction on the main body part 11 side.

More precisely, in the locked state produced by the locking mechanism 20, as shown in Fig. 6A, the hinge 21 is in a steady state without being elastically deformed, and the lock pin 21a provided at the tip of the hinge 21 is latched in the opening 12a of the battery cover 12.

With the blood glucose meter 10 of this embodiment, in a state in which the battery cover 12 is locked with respect to the main body part 11 by the locking mechanism 20, if there is an attempt to forcibly slide open the battery cover 12, the opening 12a in the battery cover 12 presses the lock pin 21a of the hinge 21 to the right in Fig. 6A. Accordingly, movement of the locked battery cover 12 is restricted, and even though a force that attempts to open the battery cover 12 is transmitted to the hinge 21, this force is applied to the hinge 21 in the compression direction. Consequently, the hinge 21 is less likely to be damaged when there is an attempt to forcibly open the battery cover 12.

Also, as shown in Fig. 12, in the locked state shown in Fig. 6 A, the end 21aa in the direction of deformation of the hinge 21 moves upward with respect to the deformation center C1 of the hinge 21, and a force acts in the direction where the lock pin 21a bites into the opening 12a and the engaging allowance becomes larger, so nothing slips out. Consequently, with this structure, the deformation direction of the lock pin 21a is controlled to make it less likely that the lock pin 21a will come out of the opening 12a, and therefore the locking force of the locking mechanism 20 can be increased.

On the other hand, in a state where the jig Z has been inserted into the opening 12a and the locking by the locking mechanism 20 has been released, as shown in Fig. 6B, a downward pressing force is applied by the jig Z, and as a result, the hinge 21 undergoes elastic deformation, and the lock pin 21a at the tip of the hinge 21 moves below the opening 12a.

At this point, the hinge 21 is held in contact with a flat receiving portion (breakage prevention portion) 11b provided to the main body part 11, as shown in Fig. 6B, in order to prevent it breakage caused by excessive force when pressed on by the jig Z and elastically deformed.

That is, when the hinge 21 is in its locked state, a gap is formed between the lower surface of the hinge 21 and the receiving portion 11b, said gap being slightly larger than the latching allowance formed in the opening 12a for the lock pin 21a.

Consequently, even if excessive force is exerted in attempting to insert the jig Z, the hinge 21 will be held by the receiving portion 11b at the position immediately after that when the lock pin 21a was removed from the opening 12a. Therefore, this prevents the hinge 21 from being broken.

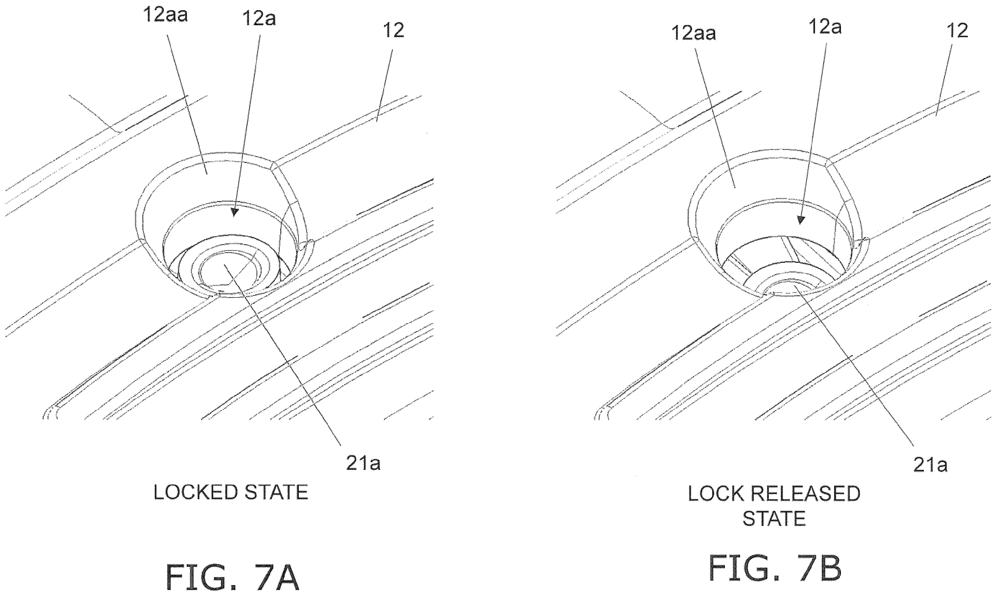

Also, in the locked state, the lock pin 21a of the locking mechanism 20 is exposed from the opening 12a, as shown in Fig. 7A.

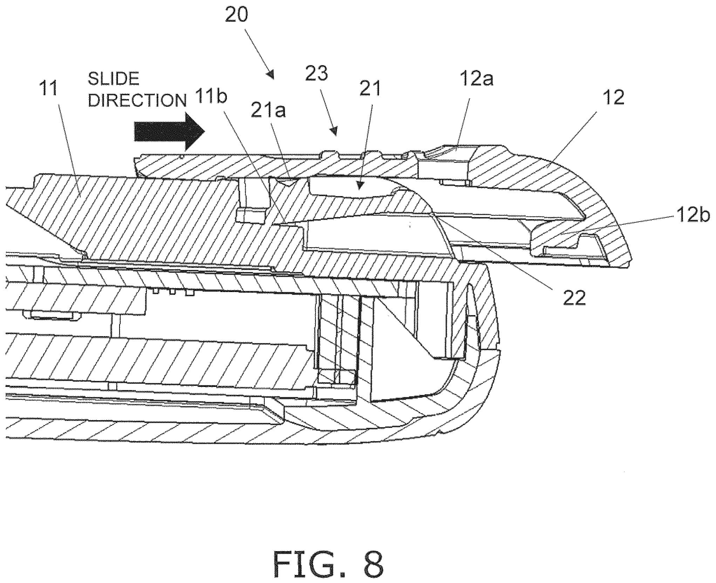

On the other hand, in the unlocked state, as shown in Fig. 7B, the lock pin 21a moves below the opening 12a, and the battery cover 12 slides as shown in Fig. 8 until it is opposite the back side of the battery cover 12.

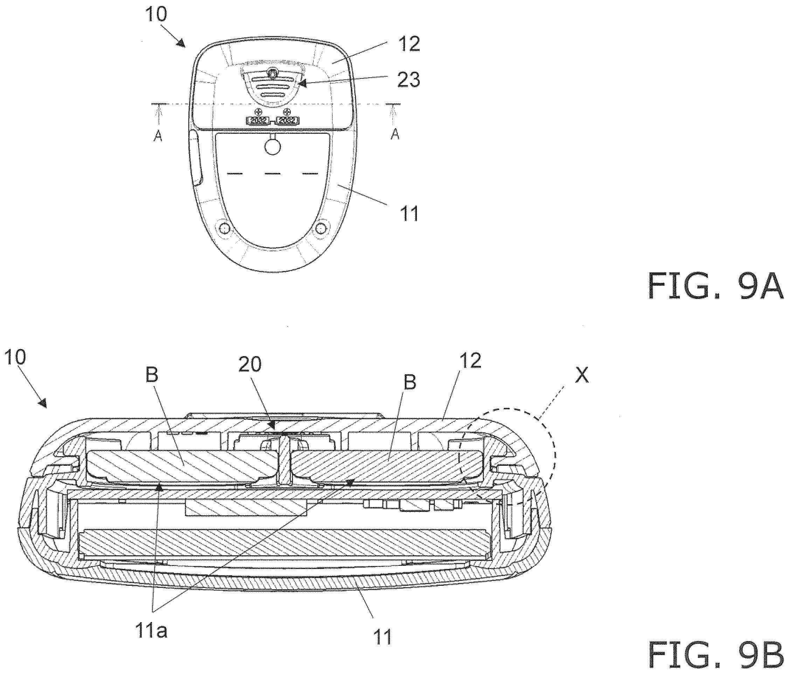

Here, the battery cover 12 is slid relative to the main body part 11 on both sides in the width direction, as shown in Figs. 9a and 9b.

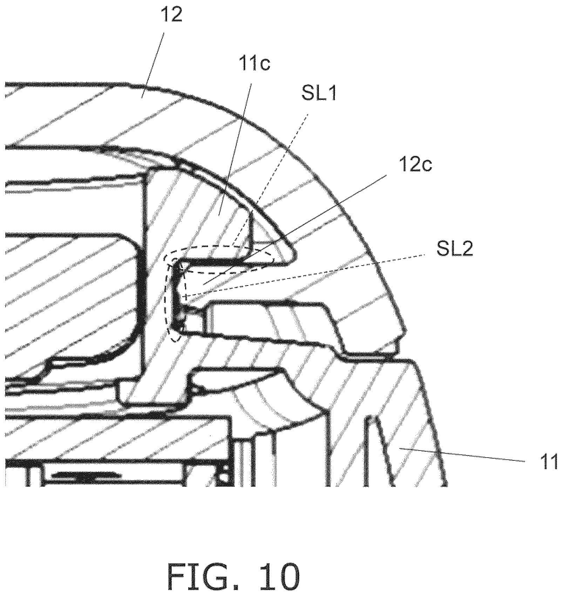

Fig. 10 is a detail view of the X portion in Fig. 9B, including the configuration on both sides in the width direction where the battery cover 12 slides with respect to the main body part 11.

That is, as shown in Fig. 10, in the battery cover 12, prongs 12c provided on both sides in the width direction are guided in the sliding direction by protrusions 11c provided on the main body part 11 side at positions opposite the prongs 12c.

At this point, as shown in Fig. 10, the prongs 12c and the protrusions 11c slide while in contact with each other over a sliding surface (slide section) SL1 running in the substantially horizontal direction and a sliding surface (slide section) SL2 running in the substantially vertical direction.

Consequently, the battery cover 12 moves in the sliding direction in a state in which movement in the lateral direction (width direction) and up and down direction (vertical direction) is restricted by the two sliding surfaces SL1 and SL2.

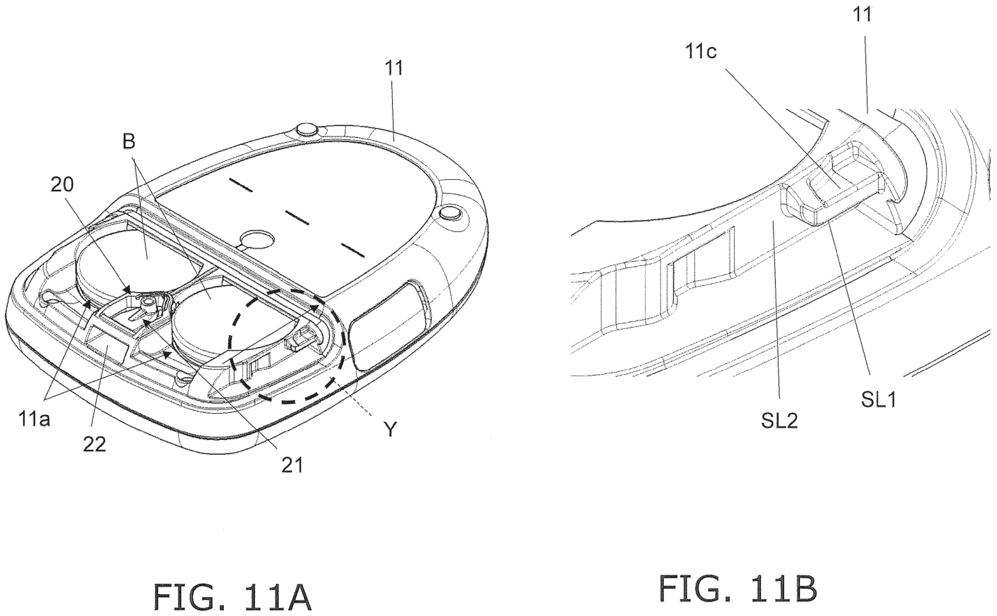

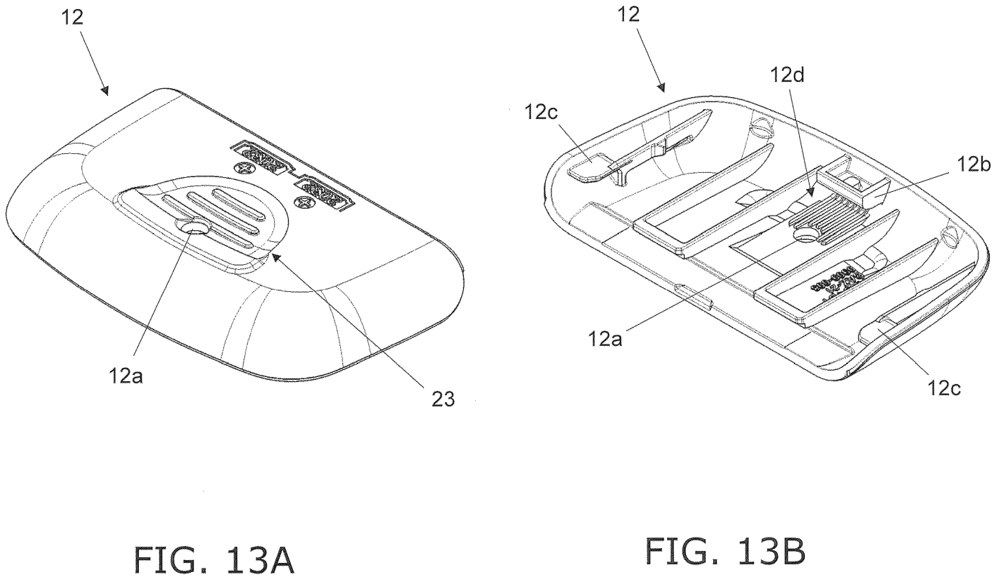

As shown in Figs. 11A and 11B, the protrusions 11c are provided over a specific length in the sliding direction. Consequently, when the prongs 12c of the battery cover 12 (see Fig. 13B) move in the sliding direction by the length of the protrusions 11c, latching in the up and down direction (vertical direction) is released and the main body part 11 is removed.

Here, as shown in Fig. 12, the lock pin 21a constituting the locking mechanism 20 is such that the first end 21aa on the side farther from the deformation center C1 of the hinge 21 in a side cross-sectional view along the lengthwise direction of the hinge 21, is higher than the second end 21ab of the hinge 21 on the side closer to the deformation center C1.

As a result, when the hinge 21 is deformed, the height of the end (first end 21aa) of the lock pin 21a located farther from the center of deformation is greater than that of the end (second end 21ab) on the opposite side (closer side), and as shown in Fig. 12, the upper surface of the lock pin 21a is inclined. Therefore, the battery cover 12 can be easily removed while ensuring a large amount of engaging allowance of the lock pin 21a with respect to the inner peripheral surface of the opening 12a of the battery cover 12 (see Fig. 12).

Also, as shown in Fig. 12, the hinge 21 has a reinforcing rib 21b that is thicker at a position close to the lock pin 21a than at a position farther away from the lock pin 21a.

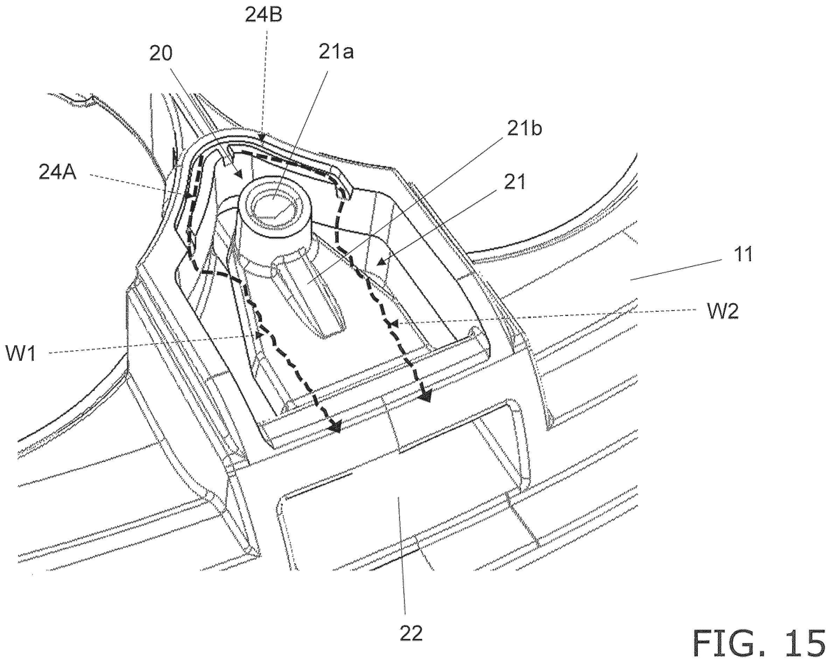

As shown in Fig. 12, the reinforcing rib 21b is formed from near the base of the lock pin 21a to just before the deformation center C1 so that the thickness is greater than that of the deformation center C1 of the hinge 21 (see Fig. 15).

This increases the rigidity of the hinge near the lock pin 21a, which prevents damage to the hinge 21 and moves the deformation center C1 to a position farther away from the lock pin 21a than in a configuration without the reinforcing rib 21b, which prevents the lock pin 21a from easily coming out of the opening 12a and releasing the locked state.

### (3) Liquid Intrusion Prevention Structure

With the blood glucose meter 10 in this embodiment, as discussed above, the opening 12a is provided to the battery cover 12, so there is the risk that blood, alcohol, water, or another such liquid will pass through the opening 12a and make its way into the interior of the main body part 11, causing the blood glucose meter 10 to malfunction. For this reason, the blood glucose meter 10 of this embodiment comprises a liquid intrusion prevention structure that guides any liquid coming through the opening 12a into the inside to the outside of the main body part 11.

More specifically, as shown in Figs. 13A and 13B, the battery cover 12 has a rib group (capillary flow path) 12d made up of a plurality of ribs on its back side.

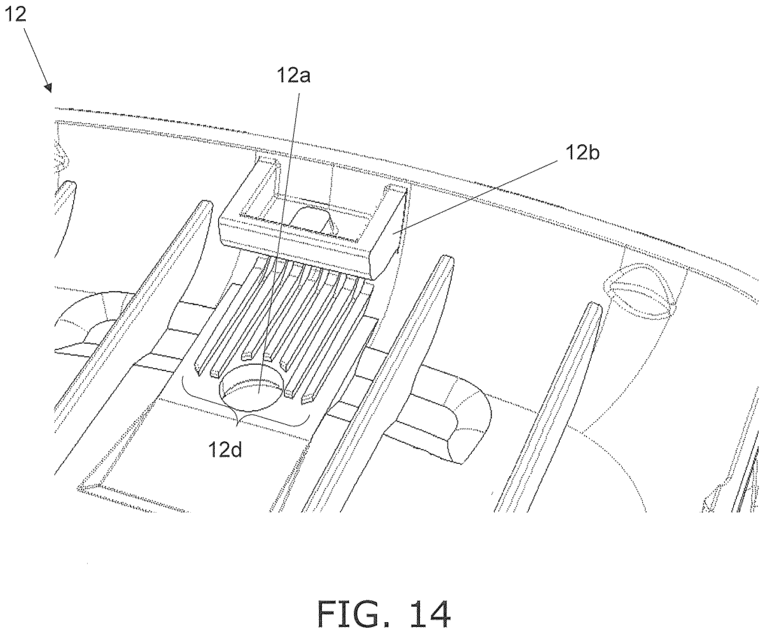

As shown in Fig. 14, the rib group 12d is formed by disposing a plurality of ribs so as to surround a semicircular portion of the opening 12a of the battery cover 12, and a capillary flow path is formed between these ribs.

The capillary flow path is provided so as to surround part of the opening 12a in the battery cover 12, and guides liquid that has come in through the opening 12a to the outside of the main body part 11 by capillary action.

On the other hand, as shown in Fig. 15, on the main body part 11 side, liquid guiding flow paths 24A and 24B are provided running around the periphery of the lock pin 21a inserted into the opening 12a.

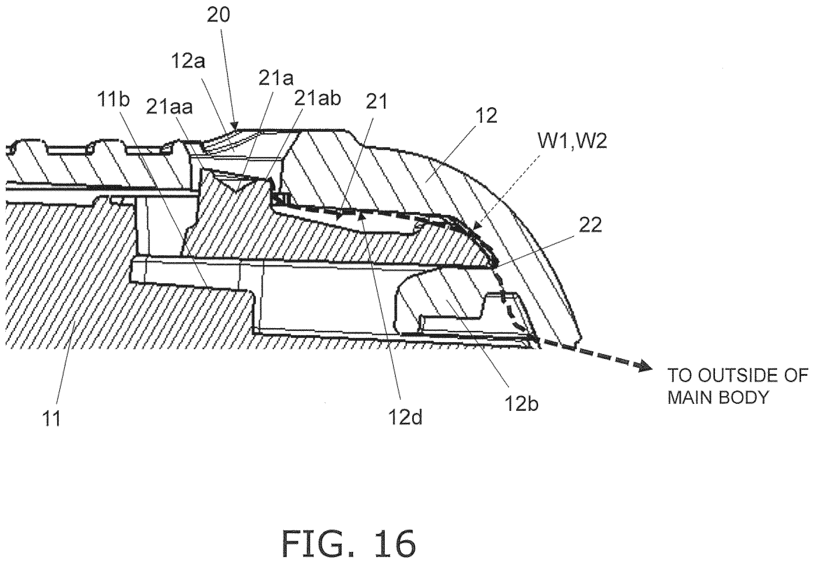

The liquid guiding flow paths 24A and 24B are provided around the lock pin 21a in the main body part 11, between the battery compartment 11a and the lock pin 21a, and divide in two at a position opposite the capillary flow path (rib group 12d) in the main body part 11. The liquid guiding flow paths 24A and 24B have grooves, making it easier to guide the liquid in the direction in which the battery cover 12 is removed (outward), which prevents the infiltration of liquid into the inside of the battery compartment 11a of the main body part 11. As shown in Fig. 16, liquid guide paths W1 and W2 indicated by dotted-line arrows guide to the outside of the main body part 11 any liquid that has moved from the opening 12a to the right side in the drawing by means of the capillary flow path (rib group 12d) formed on the back side of the battery cover 12.

Consequently, the liquid that has been guided in the desired direction by the capillary flow path (rib group 12d) provided to the battery cover 12 and by the liquid guiding flow paths 24A and 24B provided to the main body part 11 is guided to the outside of the main body part 11 along the liquid guide paths W1 and W2 while following along the main body part 11 and the battery cover 12. This prevents the liquid that has come in through the opening 12a of the battery cover 12 from moving to the battery compartment 11a in the main body part 11 and causing problems.

The liquid guiding flow paths 24A and 24B may be formed as minute gaps so that capillary action acts on the liquid, similarly to the capillary flow paths (rib group 12d).

In this case, the liquid that has moved to the liquid guiding flow paths 24A and 24B can be effectively moved to the outside of the main body part 11 by capillary action.

Also, the positional relation between the capillary flow path (rib group) 12d of the battery cover 12 and the liquid guiding flow paths (grooves) 24A and 24B of the main body part 11 may be such that these components only partially face each other, or such that they do not face each other at all, so long as the configuration prevents the liquid from coming into the battery compartment 11a.

Also, the configuration here is such that the liquid guiding flow paths 24A and 24B provided to the main body part 11 are divided in two near the middle between the two button batteries B, but the grooves may be divided into three or more parts, or may not be divided at all, so long as the configuration prevents liquid from coming into the battery compartment 11a.

Also, in this embodiment, the liquid guide paths W1 and W2 are provided only on the upper side of the hinge 21, as shown in Figs. 15 and 16, but they may also be provided on the lower side of the hinge 21.

That is, how liquid enters from the opening 12a will vary greatly depending on how the measurement subject handles the blood glucose meter 10, and the amount, speed, and angle of the liquid infiltration will also be different. If a large amount of liquid suddenly comes in, the liquid will overflow from the grooves of the liquid guiding flow paths 24A and 24B, run along the surface of the hinge 21 and the receiving portion 11b, find its way to the lower side of the hinge 21, and reach all the way to the bottom of the latching hole 22. Then, it will go through the liquid guide paths W1 and W2 on the lower side of the hinge 21, quickly run along the bottom surface of the latching hole 22, and be led in the direction of removing the battery cover 112 (outward) (not shown). At this point, even if some of the remaining liquid should pool in the space of the latching hole 22, the amount is so small that it quickly evaporates, and therefore the liquid can be prevented from getting into the battery compartment 11a.

### Main Features

The blood glucose meter 10 of this embodiment comprises the main body part 11, the sensor mounting part 13, the measurement unit 14, the battery compartment 11a, the battery cover 12, the hinge 21, the lock pin 21a, and the sliding surfaces SL1 and SL2. The sensor mounting part 13 is provided to the main body part 11, and a blood glucose level measurement sensor is mounted thereon. The measurement unit 14 measures blood deposited on the blood glucose level measurement sensor mounted in the sensor mounting part 13. The battery compartment 11a holds button batteries B that supply power to the measurement unit 14. The battery cover 12 is removably attached to the main body part 11 so as to cover the battery compartment 11a, and has the opening 12a into which the jig Z is inserted during removal from the main body part 11. The hinge 21 is provided at a position opposite the battery cover 12 in the main body part 11, and is deformed by being pressed by the jig Z inserted through the opening 12a. The lock pin 21a is provided at a position exposed to the outside through the opening 12a, and is molded integrally with the hinge 21, and when the lock pin 21a is pressed by the jig Z, the battery cover 12 is unlatched from the main body part 11. The sliding surfaces SL1 and SL2 are provided to the main body part 11, and when the latching of the lock pin 21a to the main body part 11 is released, these surfaces are slid so as to guide the battery cover 12 in the direction of removal. When the battery cover 12 is slid while still latched to the main body part 11 by the lock pin 21a, a compressive force is exerted on the hinge 21.

Consequently, when opening the battery cover 12, two operations are required: the operation of pressing the lock pin 21a from the opening 12a with the jig Z to release the lock, and the operation of sliding the battery cover 12, and this prevents a child or the like from accidentally removing the battery cover 12, for example.

Furthermore, if an attempt is made to forcibly slide the battery cover 12 while it is still locked by the lock pin 21a, a force acts on the hinge 21 in the compression direction. Therefore, if an attempt is made to forcibly slide the battery cover 12 relative to the main body part 11 while still locked by the lock pin 21a, the hinge 21 is less likely to be damaged.

As a result, a blood glucose meter 10 with which the hinge 21 is less likely to be damaged, while a child is prevented from easily taking off the battery cover 12, for example, can be provided.

### Embodiment 2

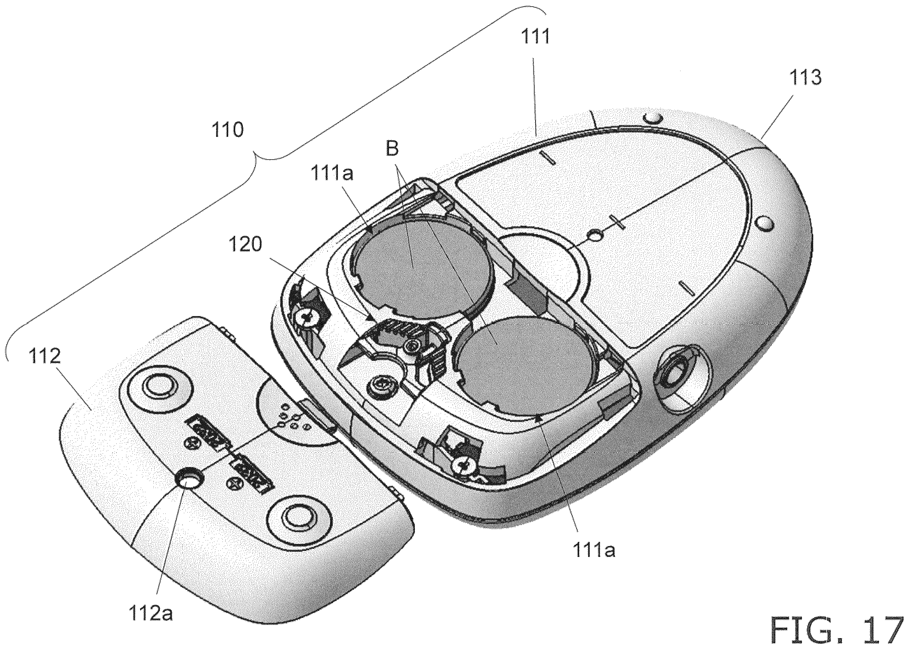

The configuration of the blood glucose meter 110 according to another embodiment of the present invention will now be described through reference to Figs. 17 to 22.

In this embodiment, those components that are the same as in the first embodiment will be numbered the same and will not be described in detail again.

As shown in Fig. 17, the blood glucose meter 110 in this embodiment comprises a main body part 111, a battery cover 112, a sensor mounting part 113, and a locking mechanism 120.

The main body part 111 has the battery cover 112 removably attached to its back side, and is provided with the measurement unit 14, the control unit 15, the button batteries (batteries) B, the storage unit 17, etc., on its inside. The main body part 111 has a battery compartment 111a into which button batteries B are installed, guide grooves 111b and 111c, and latching prongs 111d.

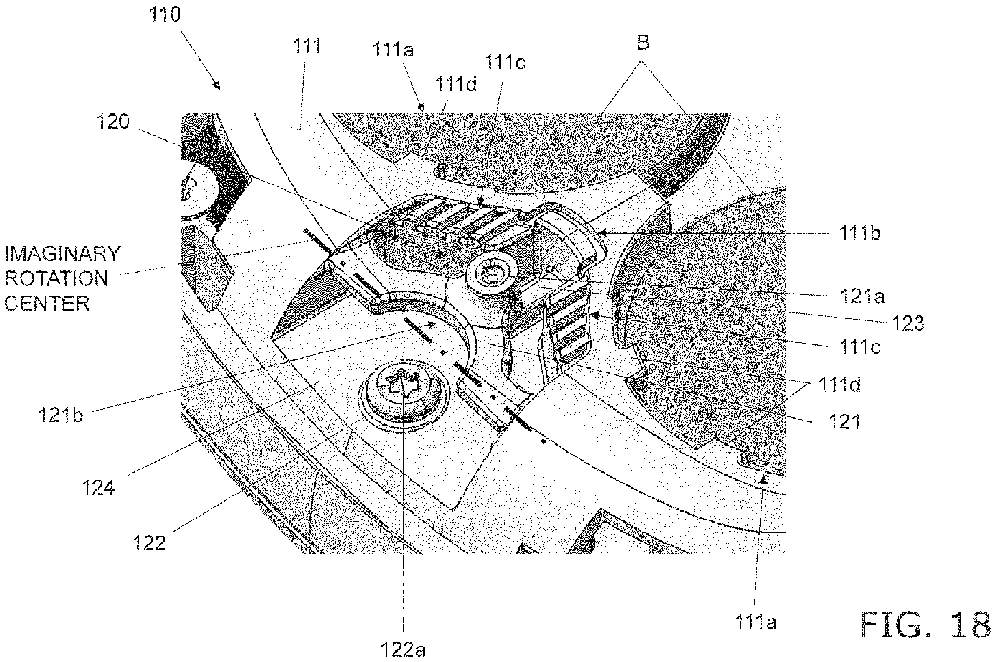

The battery compartment 111a is electrically connected to the control unit 15, and button batteries B are installed as a power source for driving the measurement unit 14, the display unit 16, and so on. Also, the battery compartment 111a has the latching prongs 111d (see Fig. 18, etc.) for preventing the installed button batteries B from falling out.

The guide grooves 111b and 111c are grooves that guide to the outside any liquid that has come into the main body part 111 through the opening 112a in the battery cover 112, and are provided around the locking mechanism 120 as shown in Fig. 18.

The guide groove 111b is a substantially U-shaped groove provided near the lock pin 121a of the hinge 121, as shown in Fig. 18, and guides the liquid that has moved along the back side of the battery cover 112 toward the guide grooves 111c.

As shown in Fig. 18, the guide grooves 111c are provided on both sides of the guide groove 111b when viewed from a side of the lock pin 121a, and are inclined downward toward the lock pin 121a. This allows the liquid that has moved to the guide grooves 111c to be guided in the direction of removing the battery cover 112 (outward).

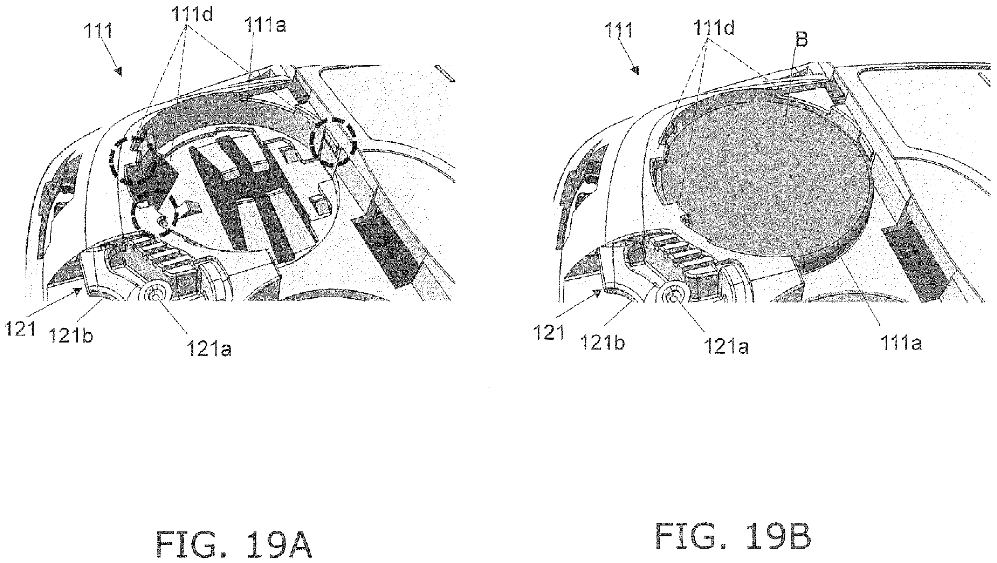

As shown in Figs. 19A and 19B, the latching prongs 111d are provided so as to protrude radially inward from the outer edge of the substantially cylindrical battery compartment 111a, and hold the button batteries B installed in the battery compartment 111a.

This effectively prevents the button batteries B from falling out of the battery compartment 111a when the battery cover 112 is removed.

The battery cover 112 is removably attached to the main body part 111 so as to cover the battery compartment 111a on the main body part 111 side, and has an opening 112a into which jig Z (see Fig. 3A etc.) is inserted during removal from the main body part 111.

The sensor mounting part 113 is provided on the end surface of the main body part 111, and a blood glucose value measurement sensor (not shown) is inserted into this. The sensor mounting part 113 is connected to the measurement unit 14, and is electrically connected to the electrodes of the blood glucose value measurement sensor.

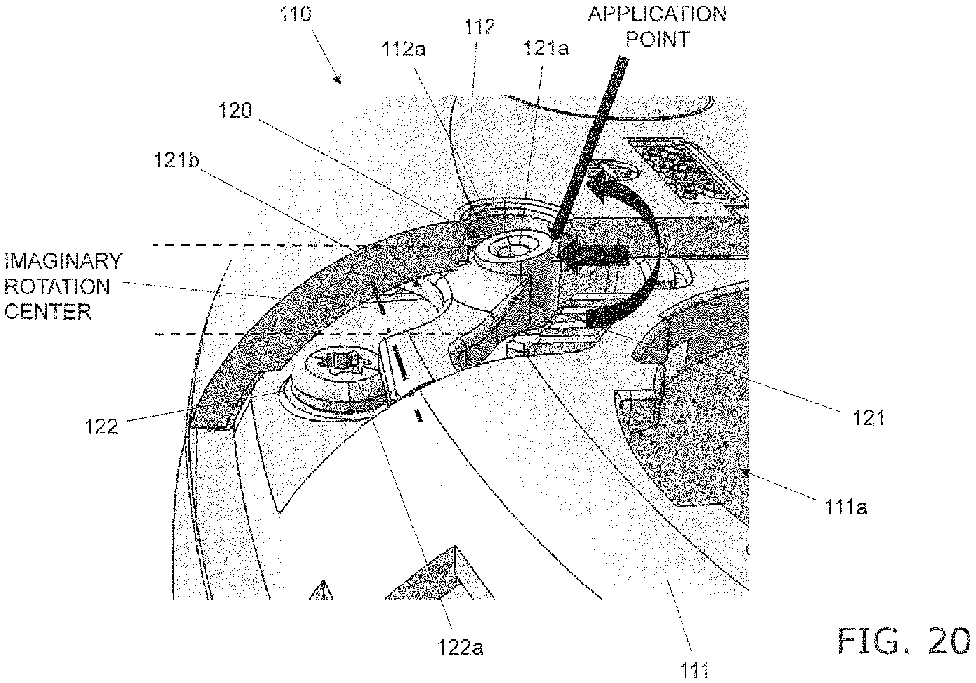

As shown in Fig. 20, the locking mechanism 120 includes a hinge (elastic deformation part) 121 provided on the main body part 111 side, and an opening 112a provided on the battery cover 112 side.

As shown in Fig. 20, the hinge 121 elastically deforms when the jig Z (not shown) is inserted into the opening 112a of the battery cover 112 and pressed downward. As a result, part of the hinge 121 (lock pin (lock portion) 121a) that was latched in the opening 112a comes out of the opening 112a, and the locked state is released. The hinge 121 has a lock pin 121a and a recess 121b.

The lock pin (lock portion) 121a is a substantially cylindrical portion integrally formed at one end of the hinge 121 so as to protrude upward in the drawing, and in the locked state restricts movement of the battery cover 112 in the sliding direction in a state in which the battery cover 112 has been inserted into the opening 112a.

As shown in Fig. 20, etc., the opening 112a is a through-hole formed in the approximate center of the battery cover 112 in the width direction, and in the locked state, the lock pin 121a is latched, and the jig Z is inserted during transition to the unlocked state.

Furthermore, just as with the opening 12a in the first embodiment described above, the smallest inside diameter of the opening 112a is smaller than the size of a child's finger.

Consequently, because an opening having a diameter smaller than about 1 cm is provided, for example, this prevents a child from accidentally unlocking the battery cover 112.

The recess 121b is formed as a depression in a part of the hinge 121 so that the part of the hinge 121 does not interfere when the screw 122a is threaded into the screw hole 122 provided for fixing the casing of the main body part 111. This prevents the hinge 121 from covering the position of the screw hole 122 in top view, allowing the screw 122a to be easily threaded into the screw hole 122.

The recess 121b is not limited to just avoiding the screw hole 122, and may be configured to avoid any interference with the hinge 121 or the liquid guide structure.

With the blood glucose meter 110 in this embodiment, if there is an attempt to remove the battery cover 112 from the main body part 111 without using the jig Z, as shown in Fig. 20, part of the inner peripheral surface of the opening 112a of the battery cover 112 pushes in the lock pin 121a of the hinge 121, so that the hinge 121 is elastically deformed around the imaginary center of rotation in the drawing.

At this point, as shown in Fig. 20, the hinge 121 is subjected to a compressive force at the point of application, and is deformed so as to be lifted upward around the imaginary center of rotation. The lock pin 121a provided at the tip of the hinge 121 then moves so as to bite into the opening 112a of the battery cover 112.

Consequently, a force acts on the hinge 121 in the compression direction, and this makes it less likely that problems such as breakage will occur as compared to when the hinge 121 is subjected to a force in the tension direction.

Also, with the blood glucose meter 110 in this embodiment, just as in Embodiment 1 above, the battery cover 112 is provided with the opening 112a, so there is a risk that blood, alcohol, water, or another such liquid may come into the blood glucose meter 110 and cause problems. Accordingly, the blood glucose meter 110 of this embodiment comprises a liquid intrusion prevention structure that guides any liquid coming through the opening 112a into the inside to the outside of the main body part 111.

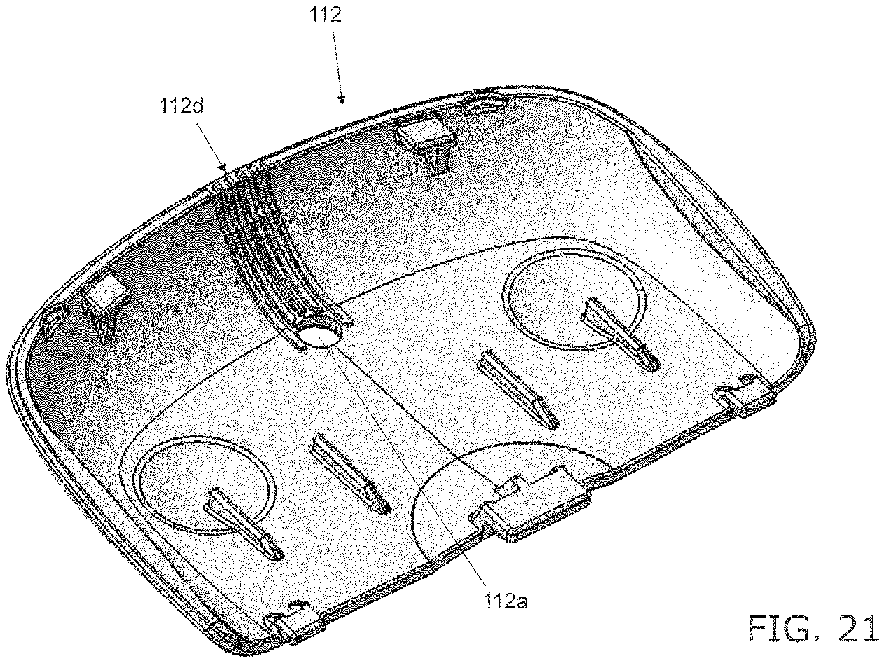

More specifically, as shown in Fig. 21, the battery cover 12 has guide grooves 112d between a rib group made up of a plurality of ribs on the back side.

As shown in Fig. 21, the guide grooves 112d are formed by disposing a plurality of ribs so as to surround a semicircular portion of the opening 112a of the battery cover 112, and a capillary flow path is formed between these ribs.

The capillary flow path is provided so as to surround part of the opening 112a in the battery cover 112, and guides liquid that has come in through the opening 112a to the outside of the main body part 111 by capillary action.

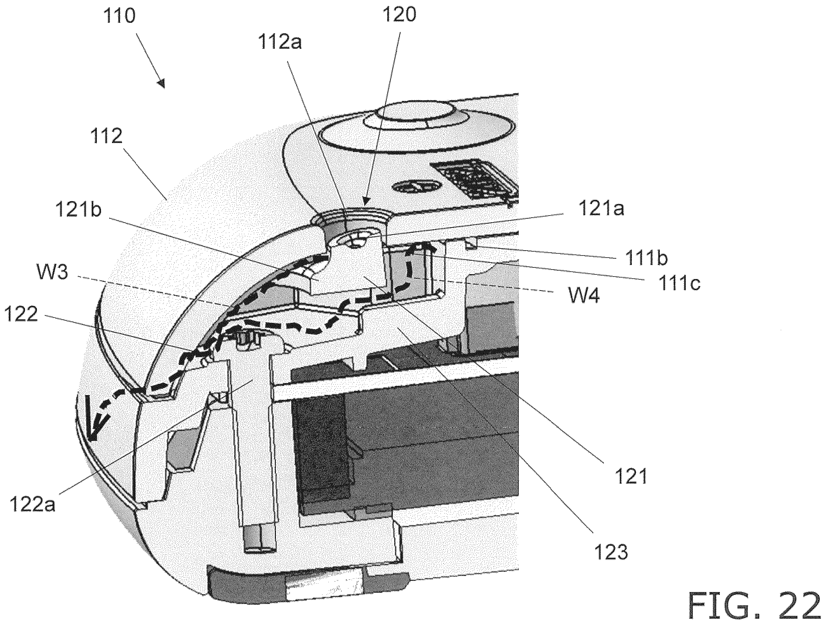

On the other hand, as shown in Figs. 18 and 22, on the main body part 111 side, guide grooves 111b and 111c are provided around the periphery of the lock pin 121a inserted into the opening 112a, and a liquid guiding flow path is formed.

The guide grooves 111b and 111c are provided around the lock pin 121a in the main body part 111, and between the battery compartment 111a and the lock pin 121a. As shown in Fig. 18, the guide grooves 111c are divided in two closer to the battery compartment 111a (on the right side in the drawing) than the position opposite the capillary flow path (guide groove 112d) in the main body part 111. The guide grooves 111b and 111c that form the liquid guiding flow path prevent the liquid from entering the battery compartment 111a of the main body part 111 by making it easier to guide the liquid in the direction in which the battery cover 112 is removed (outward).

As shown in Fig. 22, the liquid guide paths W3 and W4 direct the liquid that has moved from the opening 112a to the left side in the drawing through the capillary flow paths (guide grooves 112d) formed on the back side of the battery cover 112, to the outside of the main body part 111 in the direction of the dotted arrow.

Consequently, the liquid guided in a desired direction by the capillary flow paths (guide grooves 112d) provided to the battery cover 112 and the guide grooves 111b and 111c provided to the main body part 111 moves along the main body part 111 and the battery cover 112 while being guided to the outside of the main body part 111, as with the liquid guide paths W3 and W4. This makes it possible to avoid problems that arise when liquid that has come in through the opening 112a of the battery cover 112 moves to the battery compartment 111a inside the main body part 111.

The guide grooves 111b and 111c may be formed as tiny gaps so that the capillary action acts on the liquid, similarly to the capillary flow path (the guide groove 112d).

In this case, any liquid that has moved to the guide grooves 111b and 111c can be effectively moved to the outside of the main body part 111 by capillary action.

Also, the positional relation between the guide grooves (plurality of ribs) 112d of the battery cover 112 and the guide grooves 111b and 111c of the main body part 111 may be such these components only partially face each other, or such that they do not face each other at all, so long as the configuration prevents the liquid from coming into the battery compartment 111a.

Also, in this embodiment, the configuration is such that the guide groove 111b provided to the main body part 111 has no divided part, and the guide groove 111c is divided in two, but the grooves may be divided into three or more parts, or may not be divided at all, so long as the configuration prevents liquid from coming into the battery compartment 111a.

Also, as shown in Fig. 22, the liquid guide path W3 indicates a guide path above the hinge 121, and the liquid guide path W4 indicates a guide path below the hinge 121. How liquid enters from the opening 112a will vary greatly depending on how the measurement subject handles the blood glucose meter 110, and the amount, speed, and angle of the liquid infiltration will also be different. If a large amount of liquid suddenly comes in, the liquid will overflow from the guide grooves 111b and 111c, run along the surface of the hinge 121 and the receiving portion 123, find its way to the lower side of the hinge 121, and reach all the way to the bottom of the latching hole 124. Then, it will go through the liquid guide path W4, run along the bottom surface of the latching hole 124, and be led in the direction of removing the battery cover 112 (outward). At this point, even if some of the remaining liquid should pool in the space of the latching hole 124, the amount is so small that it quickly evaporates, and therefore the liquid can be prevented from getting into the battery compartment 111a.

### Other Embodiments

An embodiment of the present invention was described above, but the present invention is not limited to or by the above embodiment, and various changes are possible without departing from the gist of the invention which is defined by the claims.
(A) In the above embodiment, an example was given in which the blood glucose meter 10 was equipped with the hinge 21, in which the lock pin 21a was integrally molded at the distal end portion as an elastic deformation part. However, the present invention is not limited to this.
   For example, the form of the elastic deformation part is not limited to that of a hinge, and may be some other form.
(B) In the above embodiment, an example was given in which a rod-shaped member with a pointed tip was used as the jig Z used for unlocking by releasing the lock pin 21a from its state of being inserted into the opening 12a. However, the present invention is not limited to this.
   For example, the battery cover may be unlocked using a member that is used as a dedicated jig in the blood glucose meter.
(C) In the above embodiment, an example was given in which the button battery B was used as the battery that was put in the blood glucose meter 10. However, the present invention is not limited to this.

For example, the blood glucose meter may have some other kind of batteries, such as AAA dry cells, or rechargeable secondary batteries.

### INDUSTRIAL APPLICABILITY

The blood glucose meter of the present invention exhibits the effect of providing a blood glucose meter that is equipped with a locking mechanism that is resistant to damage, while preventing children from easily removing the battery cover, for example, and as such is widely applicable to various blood glucose measurement devices for measuring blood glucose levels.

### REFERENCE SIGNS LIST

10 blood glucose meter
11 main body part
11a battery compartment
11b receiving portion (damage prevention part)
11c protrusion
12 battery cover
12a opening
12aa C surface (chamfered part)
12b latched part
12c prong
12d rib group (capillary flow path)
13 sensor mounting part
14 measurement unit
15 control unit
16 display unit
17 storage unit
20 locking mechanism
21 hinge (elastic deformation part)
21a lock pin (lock portion)
21aa first end
21ab second end
21b reinforcing rib
22 latching hole
23 finger pad
24A, 24B liquid guiding flow path (groove)
110 blood glucose meter
111 main body part
111a battery compartment
111b guide groove
111c guide groove
111d latching prong
112 battery cover
112a opening
112d guide groove
113 sensor mounting part
120 locking mechanism
121 hinge (elastic deformation part)
121a lock pin (lock portion)
121b recess
122 screw hole
122a screw
123 receiving portion
124 latching hole
B button battery (battery)
C1 deformation center
SL1, SL2 sliding surface (slide section)
W1, W2, W3, W4 liquid guide path
Z jig

## Claims

1. A blood glucose meter (10, 110), comprising:
a main body part (11, 111);
a sensor mounting part (13, 113) that is provided to the main body part (11, 111) and to which a blood glucose value measurement sensor is mounted;
a measurement unit (14) configured to measure blood deposited on the blood glucose value measurement sensor mounted on the sensor mounting part (13, 113);
a battery compartment (11a, 111a) that holds a battery (B) configured to supply power to the measurement unit (14);
a battery cover (12, 112) that is removably attached to the main body part (11, 111) so as to cover the battery compartment (11a, 111a), and has an opening (12a, 112a) into which a jig (Z) is inserted during removal from the main body part (11, 111);
an elastic deformation part (21, 121) that is provided at a position opposite the battery cover (12, 112) in the main body part (11, 111) and is configured to deform when pressed by the jig (Z) inserted through the opening (12a, 112a);
a lock portion (21a, 121a) that is provided at a position exposed to an outside from the opening (12a, 112a), is molded integrally with the elastic deformation part (21, 121), and is configured to unlatch the battery cover (12, 112) from the main body part (11, 111) when pressed by the jig (Z); and
a slide part (SL1, SL2) that is provided to the main body part (11, 111) and is configured to slide the battery cover (12, 112) so as to guide the battery cover (12, 112) in a removal direction when the lock portion (21a, 121a) is unlatched from the main body part (11, 111),
wherein when the battery cover (12, 112) is slid along the slide part (SL1, SL2) while the battery cover (12, 112) remains latched to the main body part (11, 111) by the lock portion (21a, 121a), a force in a compression direction is exerted on the elastic deformation part (21, 121).

2. The blood glucose meter (10, 110) according to claim 1,
further comprising a capillary flow path (12d, 112d) that is provided so as to surround at least part of the opening (12a, 112a) in the battery cover (12, 112), and that is configured to guide liquid that has entered through the opening (12a, 112a) by capillary action to the outside of the main body part (11, 111).

3. The blood glucose meter (10, 110) according to claim 2,
wherein the capillary flow path (12d, 112d) is formed as a groove (12d, 112d) provided between a plurality of ribs (12d, 112d) provided around the opening (12a, 112a).

4. The blood glucose meter (10, 110) according to claim 2 or 3,
wherein the capillary flow path (12d, 112d) is provided on a rear surface side of the battery cover (12, 112).

5. The blood glucose meter (10, 110) according to any of claims 2 to 4,
further comprising a liquid guiding flow path (24A, 24B, W1, W2, W3, W4) that is provided to the main body part (11, 111) and is configured to guide the liquid that has come in through the opening (12a, 112a) to an outside of the main body part (11, 111).

6. The blood glucose meter (10, 110) according to claim 5,
wherein the liquid guiding flow path (24A, 24B, W1, W2, W3, W4) is provided to the main body part (11, 111) at a position opposite the capillary flow path (12d, 112d).

7. The blood glucose meter (10, 110) according to any of claims 1 to 6, further comprising:
a latching hole (22, 124) that is provided to the main body part (11, 111) and is formed along the sliding direction of the battery cover (12, 112); and
a latched part (12b) that is provided to the battery cover (12, 112), and is inserted into the latching hole (22, 124) and is configured to restrict movement in a direction intersecting the sliding direction when the battery cover (12, 112) is attached to the main body part (11, 111).

8. The blood glucose meter (10, 110) according to any of claims 1 to 7,
wherein the lock portion (21a, 121a) is formed such that an end that is farther from a center of deformation (C1) when the jig (Z) is inserted into the opening (12a, 112a) and the elastic deformation part (21, 121) is deformed is higher than an end that is closer to the center of deformation (C1).

9. The blood glucose meter (10, 110) according to any of claims 1 to 8,
wherein the elastic deformation part (21, 121) has a reinforcing rib (21b) that is provided at a position close to the lock portion (21a, 121a) and has a thicker wall than a position farther away from the lock portion (21a, 121a).

10. The blood glucose meter (10, 110) according to any of claims 1 to 9,
wherein the lock portion (21a, 121a) is provided at a position farther to an inside than an outer contour of the main body part (11, 111), through the opening (12a, 112a).

11. The blood glucose meter (10, 110) according to any of claims 1 to 10,
wherein the opening (12a, 112a) has a chamfered portion (12aa) whose diameter increases, at an upper end in an insertion direction of the jig (Z).

12. The blood glucose meter (10, 110) according to any of claims 1 to 11,
wherein the opening (12a, 112a) has a diameter smaller than a size of a child's finger.

13. The blood glucose meter (10, 110) according to claim 1 or 2,
wherein the elastic deformation part (21, 121) has a recess (121b) formed so as to avoid the position of a screw (122a) attached to the main body part (11, 111).

14. The blood glucose meter (10, 110) according to claim 1 or 2,
wherein the main body part (11, 111) has a latching prong (111d) that protrudes from an outer peripheral side of the battery compartment (11a, 111a) and holds the battery (B) housed in the battery compartment (11a, 111a).

15. The blood glucose meter (10, 110) according to claim 1 or 2,
wherein the elastic deformation part (21, 121) is fixed to the main body part (11, 111) at a plurality of positions that are away from the lock portion (21a, 121a).

## Patentansprüche

1. Blutzuckermessgerät (10, 110), umfassend:
einen Hauptteil (11, 111);
ein Sensoranbringungsteil (13, 113), das an dem Hauptteil (11, 111) vorgesehen ist und an dem ein Blutzuckerwert-Messsensor angebracht ist;
eine Messeinheit (14), die dazu ausgestaltet ist, Blut zu messen, das auf den an dem Sensoranbringungsteil (13, 113) angebrachten Blutzuckerwert-Messsensor aufgetragen ist;
ein Batteriefach (11a, 111a), das eine Batterie (B) aufnimmt, die dazu ausgestaltet ist, die Messeinheit (14) mit Strom zu versorgen;
einen Batteriedeckel (12, 112), der abnehmbar an dem Hauptteil (11, 111) angebracht ist, um das Batteriefach (11a, 111a) abzudecken, und der eine Öffnung (12a, 112a) aufweist, in die beim Entfernen von dem Hauptteil (11, 111) ein Werkzeug (Z) eingeführt wird;
ein elastisches Verformungsteil (21, 121), das an einer dem Batteriedeckel (12, 112) gegenüberliegenden Position in dem Hauptteil (11, 111) vorgesehen ist und dazu ausgestaltet ist, sich zu verformen, wenn es durch das durch die Öffnung (12a, 112a) eingeführte Werkzeug (Z) gedrückt wird;
ein Verriegelungsteil (21a, 121a), das an einer von der Öffnung (12a, 112a) nach außen freiliegenden Position vorgesehen ist, einstückig mit dem elastischen Verformungsteil (21, 121) ausgestaltet ist und dazu ausgestaltet ist, den Batteriedeckel (12, 112) von dem Hauptteil (11, 111) zu entriegeln, wenn es durch das Werkzeug (Z) gedrückt wird; und
ein Gleitteil (SL1, SL2), das an dem Hauptteil (11, 111) vorgesehen ist und dazu ausgestaltet ist, den Batteriedeckel (12, 112) gleiten zu lassen, um den Batteriedeckel (12, 112) in eine Entnahmerichtung zu führen, wenn das Verriegelungsteil (21a, 121a) gegenüber dem Hauptteil (11, 111) entriegelt ist,
wobei, wenn der Batteriedeckel (12, 112) entlang dem Gleitteil (SL1, SL2) verschoben wird, während der Batteriedeckel (12, 112) durch das Verriegelungsteil (21a, 121a) an dem Hauptteil (11, 111) eingerastet bleibt, eine Kraft in einer Druckrichtung auf das elastische Verformungsteil (21, 121) ausgeübt wird.

2. Blutzuckermessgerät (10, 110) nach Anspruch 1, ferner mit einem Kapillarströmungspfad (12d, 112d), der so vorgesehen ist, dass er zumindest einen Teil der Öffnung (12a, 112a) in dem Batteriedeckel (12, 112) umgibt, und der dazu ausgestaltet ist, durch die Öffnung (12a, 112a) eingedrungene Flüssigkeit durch Kapillarwirkung nach außerhalb des Hauptteils (11, 111) zu führen.

3. Blutzuckermessgerät (10, 110) nach Anspruch 2, wobei der Kapillarströmungspfad (12d, 112d) als eine Rille (12d, 112d) ausgestaltet ist, die zwischen mehreren Rippen (12d, 112d) vorgesehen ist, die um die Öffnung (12a, 112a) angeordnet sind.

4. Blutzuckermessgerät (10, 110) nach Anspruch 2 oder 3, wobei der Kapillarströmungspfad (12d, 112d) auf einer Rückseite des Batteriedeckels (12, 112) vorgesehen ist.

5. Blutzuckermessgerät (10, 110) nach einem der Ansprüche 2 bis 4, ferner mit einem Flüssigkeitsführungsströmungspfad (24A, 24B, W1, W2, W3, W4), der an dem Hauptteil (11, 111) vorgesehen ist und dazu ausgestaltet ist, die durch die Öffnung (12a, 112a) eingedrungene Flüssigkeit nach außerhalb des Hauptteils (11, 111) zu führen.

6. Blutzuckermessgerät (10, 110) nach Anspruch 5, wobei der Flüssigkeitsführungsströmungspfad (24A, 24B, W1, W2, W3, W4) an dem Hauptteil (11, 111) an einer dem Kapillarströmungspfad (12d, 112d) gegenüberliegenden Position vorgesehen ist.

7. Blutzuckermessgerät (10, 110) nach einem der Ansprüche 1 bis 6, ferner umfassend:
ein Sperrloch (22, 124), das an dem Hauptteil (11, 111) vorgesehen ist und entlang der Gleitrichtung des Batteriedeckels (12, 112) gebildet ist; und
einem Sperrteil (12b), das an dem Batteriedeckel (12, 112) vorgesehen ist und in das Sperrloch (22, 124) eingeführt ist und dazu ausgestaltet ist, eine Bewegung in einer die Gleitrichtung schneidenden Richtung zu beschränken, wenn der Batteriedeckel (12, 112) an dem Hauptteil (11, 111) angebracht ist.

8. Blutzuckermessgerät (10, 110) nach einem der Ansprüche 1 bis 7, wobei das Verriegelungsteil (21a, 121a) so ausgestaltet ist, dass ein Ende, das weiter von einem Verformungszentrum (C1) entfernt ist, wenn das Werkzeug (Z) in die Öffnung (12a, 112a) eingeführt und das elastische Verformungsteil (21, 121) verformt wird, höher ist als ein Ende, das näher an dem Verformungszentrum (C1) liegt.

9. Blutzuckermessgerät (10, 110) nach einem der Ansprüche 1 bis 8, wobei das elastische Verformungsteil (21, 121) eine Verstärkungsrippe (21b) aufweist, die an einer Position nahe dem Verriegelungsteil (21a, 121a) vorgesehen ist und eine dickere Wandung aufweist als eine Position, die weiter von dem Verriegelungsteil (21a, 121a) entfernt ist.

10. Blutzuckermessgerät (10, 110) nach einem der Ansprüche 1 bis 9, wobei das Verriegelungsteil (21a, 121a) an einer Position vorgesehen ist, die durch die Öffnung (12a, 112a) weiter innen liegt als eine Außenkontur des Hauptteils (11, 111).

11. Blutzuckermessgerät (10, 110) nach einem der Ansprüche 1 bis 10, wobei die Öffnung (12a, 112a) an einem oberen Ende in einer Einführrichtung des Werkzeugs (Z) einen angeschrägten Teil (12aa) aufweist, dessen Durchmesser sich vergrößert.

12. Blutzuckermessgerät (10, 110) nach einem der Ansprüche 1 bis 11, wobei die Öffnung (12a, 112a) einen Durchmesser aufweist, der kleiner ist als die Größe eines Kinderfingers.

13. Blutzuckermessgerät (10, 110) nach Anspruch 1 oder 2, wobei das elastische Verformungsteil (21, 121) eine Vertiefung (121b) aufweist, die so ausgestaltet ist, dass sie die Position einer an dem Hauptteil (11, 111) angebrachten Schraube (122a) umgeht.

14. Blutzuckermessgerät (10, 110) nach Anspruch 1 oder 2, wobei der Hauptteil (11, 111) einen Verriegelungsvorsprung (111d) aufweist, der von einer äußeren Umfangsseite des Batteriefachs (11a, 111a) vorsteht und die in dem Batteriefach (11a, 111a) untergebrachte Batterie (B) hält.

15. Blutzuckermessgerät (10, 110) nach Anspruch 1 oder 2, wobei das elastische Verformungsteil (21, 121) an dem Hauptteil (11, 111) an mehreren Positionen fixiert ist, die von dem Verriegelungsteil (21a, 121a) entfernt sind.

## Revendications

1. Lecteur de glycémie (10, 110) comprenant:
une partie de corps principal (11, 111);
une partie de montage de capteur (13, 113) qui est prévue sur la partie de corps principal (11, 111) et sur laquelle est monté un capteur de mesure de valeur de glycémie;
une unité de mesure (14) qui est configurée pour mesurer du sang déposé sur le capteur de mesure de valeur de glycémie qui est monté sur la partie de montage de capteur (13, 113);
un compartiment de batterie (11a, 111a) qui loge une batterie (B) laquelle est configurée pour alimenter en courant l'unité de mesure (14);
un couvercle de batterie (12, 112) qui est fixé de manière amovible à la partie de corps principal (11, 111) de façon à recouvrir le compartiment de batterie (11a, 111a), et qui comprend une ouverture (12a, 112a) dans laquelle un outil (Z) est inséré lors du retrait de la partie de corps principal (11, 111);
une partie de déformation élastique (21, 121) qui est prévue au niveau d'une position opposée au couvercle de batterie (12, 112) dans la partie de corps principal (11, 111) et est configurée pour se déformer lorsqu'elle est pressée par l'outil (Z) inséré à travers l'ouverture (12a, 112a);
une partie de verrouillage (21a, 121a) qui est prévue au niveau d'une position exposée à un extérieur depuis l'ouverture (12a, 112a), est moulée d'un seul tenant avec la partie de déformation élastique (21, 121), et est configurée pour déverrouiller le couvercle de batterie (12, 112) de la partie de corps principal (11, 111) lorsqu'elle est pressé par l'outil (Z) ; et
une partie coulissante (SL1, SL2) qui est prévue sur la partie de corps principal (11, 111) et est configurée pour faire coulisser le couvercle de batterie (12, 112) de manière à guider le couvercle de batterie (12, 112) dans une direction de retrait lorsque la partie de verrouillage (21a, 121a) est déverrouillée de la partie de corps principal (11, 111),
dans lequel, lorsque le couvercle de batterie (12, 112) coulisse le long de la partie coulissante (SL1, SL2) tandis que le couvercle de batterie (12, 112) reste verrouillé sur la partie de corps principal (11, 111) par la partie de verrouillage (21a, 121a), une force dans une direction de compression est exercée sur la partie de déformation élastique (21, 121).

2. Lecteur de glycémie (10, 110) selon la revendication 1,
comprenant en outre un chemin d'écoulement capillaire (12d, 112d) qui est prévu de manière à entourer au moins une partie de l'ouverture (12a, 112a) dans le couvercle de batterie (12, 112), et qui est configuré pour guider du liquide qui est entré par l'ouverture (12a, 112a) par action capillaire vers l'extérieur de la partie de corps principal (11, 111).

3. Lecteur de glycémie (10, 110) selon la revendication 2,
dans lequel le chemin d'écoulement capillaire (12d, 112d) est formé sous la forme d'une rainure (12d, 112d) qui est prévue entre une pluralité de nervures (12d, 112d) prévues autour de l'ouverture (12a, 112a).

4. Lecteur de glycémie (10, 110) selon la revendication 2 ou 3,
dans lequel le chemin d'écoulement capillaire (12d, 112d) est prévu sur un côté de surface arrière du couvercle de batterie (12, 112).

5. Lecteur de glycémie (10, 110) selon l'une quelconque des revendications 2 à 4,
comprenant en outre un chemin d'écoulement de guidage de liquide (24A, 24B, W1, W2, W3, W4) qui est prévu sur la partie de corps principal (11, 111) et est configuré pour guider le liquide qui est entré par l'ouverture (12a, 112a) vers un extérieur de la partie de corps principal (11, 111).

6. Lecteur de glycémie (10, 110) selon la revendication 5,
dans lequel le chemin d'écoulement de guidage de liquide (24A, 24B, W1, W2, W3, W4) est prévu sur la partie de corps principal (11, 111) au niveau d'une position opposée au chemin d'écoulement capillaire (12d, 112d).

7. Lecteur de glycémie (10, 110) selon l'une quelconque des revendications 1 à 6, comprenant en outre:
un trou de verrouillage (22, 124) qui est prévu sur la partie de corps principal (11, 111) et est formé le long de la direction de coulissement du couvercle de batterie (12, 112); et
une partie verrouillée (12b) qui est prévue sur le couvercle de batterie (12, 112), et est insérée dans le trou de verrouillage (22, 124) et est configurée pour restreindre un mouvement dans une direction coupant la direction de coulissement lorsque le couvercle de batterie (12, 112) est fixé à la partie de corps principal (11, 111).

8. Lecteur de glycémie (10, 110) selon l'une quelconque des revendications 1 à 7,
dans lequel la partie de verrouillage (21a, 121a) est formée de telle sorte qu'une extrémité qui est plus éloignée d'un centre de déformation (C1) lorsque l'outil (Z) est inséré dans l'ouverture (12a, 112a) et que la partie de déformation élastique (21, 121) est déformée est plus haute qu'une extrémité qui est plus proche du centre de déformation (C1).

9. Lecteur de glycémie (10, 110) selon l'une quelconque des revendications 1 à 8,
dans lequel la partie de déformation élastique (21, 121) comprend une nervure de renforcement (21b) qui est prévue au niveau d'une position proche de la partie de verrouillage (21a, 121a) et présente une paroi plus épaisse qu'une position plus éloignée de la partie de verrouillage (21a, 121a).

10. Lecteur de glycémie (10, 110) selon l'une quelconque des revendications 1 à 9,
dans lequel la partie de verrouillage (21a, 121a) est prévue au niveau d'une position plus à l'intérieur qu'un contour externe de la partie de corps principale (11, 111), à travers l'ouverture (12a, 112a).

11. Lecteur de glycémie (10, 110) selon l'une quelconque des revendications 1 à 10,
dans lequel l'ouverture (12a, 112a) comprend, au niveau d'une extrémité supérieure dans une direction d'insertion de l'outil (Z), une partie chanfreinée (12aa) dont le diamètre augmente.

12. Lecteur de glycémie (10, 110) selon l'une quelconque des revendications 1 à 11,
dans lequel l'ouverture (12a, 112a) présente un diamètre inférieur à la taille du doigt d'un enfant.

13. Lecteur de glycémie (10, 110) selon la revendication 1 ou 2,
dans lequel la partie de déformation élastique (21, 121) comprend un évidement (121b) qui est formé de manière à éviter la position d'une vis (122a) fixée à la partie de corps principal (11, 111).

14. Lecteur de glycémie (10, 110) selon la revendication 1 ou 2,
dans lequel la partie de corps principal (11, 111) comprend un ergot de verrouillage (111d) qui fait saillie depuis un côté périphérique externe du compartiment de batterie (11a, 111a) et maintient la batterie (B) logée dans le compartiment de batterie (11a, 111a).

15. Lecteur de glycémie (10, 110) selon la revendication 1 ou 2,
dans lequel la partie de déformation élastique (21, 121) est fixée à la partie de corps principal (11, 111) au niveau d'une pluralité de positions qui sont éloignées de la partie de verrouillage (21a, 121a).
